(19) Europäisches Patentamt
European Patent Office
Office européen des brevets

(11) EP 1 314 425 A1

(12) **EUROPEAN PATENT APPLICATION**
published in accordance with Art. 158(3) EPC

(43) Date of publication:
**28.05.2003 Bulletin 2003/22**

(21) Application number: **01963398.1**

(22) Date of filing: **29.08.2001**

(51) Int Cl.7: **A61K 31/404**, A61K 31/22, A61K 31/366, A61K 31/41, A61K 31/4184, A61K 31/505, A61K 31/47, A61K 45/06, A61P 9/10, A61P 9/04, A61P 43/00

(86) International application number:
**PCT/JP01/07437**

(87) International publication number:
**WO 02/017913 (07.03.2002 Gazette 2002/10)**

(84) Designated Contracting States:
**AT BE CH CY DE DK ES FI FR GB GR IE IT LI LU MC NL PT SE TR**

(30) Priority: **30.08.2000 JP 2000260949**

(71) Applicant: **Sankyo Company, Limited**
**Tokyo 103-8426 (JP)**

(72) Inventors:
- **LEE, Tsung, Ming**
  **Taipei-city (TW)**
- **LEE, Bai-Ching**
  **Chung-Ho city, Taipei (TW)**
- **SU, Shen-Fang**
  **Tainan-city (TW)**
- **HSIAO, Chia-Ling**
  **Chung-ho city, Taipei (TW)**
- **CHU, Chia-Wei**
  **Kaoshung-city (TW)**

(74) Representative:
**Gibson, Christian John Robert et al**
**MARKS & CLERK,**
**57/60 Lincoln's Inn Fields**
**London WC2A 3LS (GB)**

# (54) MEDICINAL COMPOSITIONS FOR PREVENTING OR TREATING HEART FAILURE

(57) The present invention relates to pharmaceutical compositions, which contain a HMG-CoA reductase inhibitor selected from the group consisting of pravastatin, simvastatin, lovastatin, pitavastatin and ZD-4522 and an angiotensin II receptor antagonist and optionally further contain a calcium channel blocker.

EP 1 314 425 A1

## Description

[Technical field]

The present invention relates to pharmaceutical compositions for the prevention or treatment of cardiac failure, the prevention of ischemic coronary heart disease or the prevention of the recurrence of ischemic coronary heart disease, said pharmaceutical compositions containing a HMG-CoA reductase inhibitor selected from the group consisting of pravastatin, simvastatin, lovastatin, pitavastatin and ZD-4522 and an angiotensin II receptor antagonist and optionally further containing a calcium channel blocker.

The present invention also relates to methods for the prevention or treatment of cardiac failure, the prevention of ischemic coronary heart disease or the prevention of the recurrence of ischemic coronary heart disease, said methods comprising administering to a warm-blooded animal (particularly a human) in need of such treatment or prevention a pharmacologically effective amount of a pharmaceutical composition that contains a HMG-CoA reductase inhibitor selected from the group consisting of pravastatin, simvastatin, lovastatin, pitavastatin and ZD-4522 and an angiotensin II receptor antagonist and optionally further contains a calcium channel blocker.

[Technical background]

HMG-CoA reductase inhibitors such as pravastatin are well known as an anti-hyperlipidemic agent (for example, U.S. Patent Nos. 4346227, 4444784, 4231938, 5856336, 5260440 and the like) and such medicaments have been placed on the market.

Angiotensin II receptor antagonists are well known as an antihypertensive agent (for example, U.S. Patent Nos. 5138069, 5196444, 5616599 and the like) and a lot of such medicaments have been placed on the market.

Calcium channel blockers are well known as an antihypertensive agent (for example, U.S. Patent Nos. 3485847, 3985758, 4572909 and the like) and a lot of such medicaments have been placed on the market.

A pharmaceutical agent containing an angiotensin II acceptor antagonist and a pyridine derivative having HMG-CoA reductase inhibitory activity (particularly for circulatory system disease, Japanese Patent Application Publication number No. Hei 9-323940), a pharmaceutical composition containing an angiotensin II acceptor antagonist and a HMG-CoA reductase inhibitor (particularly a drug for arteriosclerosis, Japanese Patent Application Publication No. Hei 10-81633), a pharmaceutical composition containing amlodipine and atorvastatin (particularly a heart disease risk factor inhibitor, WO Publication No. 99/11259), a pharmaceutical composition containing atorvastatin and an antihypertensive agent (particularly a heart disease risk factor inhibitor, WO Publication No. 99/11260), a pharmaceutical composition containing amlodipine and a statin derivative (particularly a heart disease risk factor inhibitor, WO Publication No. 99/11263) and the like are known as combination agents comprising these medicaments.

A pharmaceutical composition for the prevention or treatment of cardiac failure, the prevention of ischemic coronary heart disease or the prevention of the recurrence of ischemic coronary heart disease, said pharmaceutical compositions containing a particular HMG-CoA reductase inhibitor such as pravastatin and an angiotensin II receptor antagonist have not previously been disclosed.

[Disclosure of the invention]

The inventors have made a great effort to study pharmaceutical compositions containing a HMG-CoA reductase inhibitor, angiotensin II receptor antagonist and/or a calcium channel blocker for many years and found that a pharmaceutical composition containing particular pharmaceutical agents exhibit excellent left ventriclar hypertrophy inhibition activity and are useful pharmaceutical compositions for the prevention or treatment of cardiac failure, the prevention of ischemic coronary heart disease or the prevention of the recurrence of ischemic coronary heart disease.

The present invention provides pharmaceutical compositions for the prevention or treatment of cardiac failure, the prevention of ischemic coronary heart disease or the prevention of the recurrence of ischemic coronary heart disease, said pharmaceutical compositions containing a HMG-CoA reductase inhibitor selected from the group consisting of pravastatin, simvastatin, lovastatin, pitavastatin and ZD-4522 and an angiotensin II receptor antagonist and optionally further containing a calcium channel blocker.

The present invention also relates to methods for the prevention or treatment of cardiac failure, the prevention of ischemic coronary heart disease or the prevention of the recurrence of ischemic coronary heart disease, said methods comprising administering to a warm-blooded animal (particularly a human) in need of such treatment or prevention a pharmacologically effective amount of a pharmaceutical composition that contains a HMG-CoA reductase inhibitor selected from the group consisting of pravastatin, simvastatin, lovastatin, pitavastatin and ZD-4522 and an angiotensin II receptor antagonist and optionally further contains a calcium channel blocker.

The pharmaceutical composition of the present invention, which is for the prevention or treatment of cardiac

failure, the prevention of ischemic coronary heart disease or the prevention of the recurrence of ischemic coronary heart disease, contains as active ingredients a HMG-CoA reductase inhibitor selected from the group consisting of pravastatin, simvastatin, lovastatin, pitavastatin and ZD-4522 and an angiotensin II receptor antagonist and optionally further contains a calcium channel blocker.

An active ingredient of the pharmaceutical composition is a HMG-CoA reductase inhibitor selected from the group consisting of pravastatin, simvastatin, lovastatin, pitavastatin and ZD-4522; preferably pravastatin, simvastatin, pitavastatin or ZD-4522; more preferably pravastatin, simvastatin or ZD-4522; still more preferably pravastatin or simvastatin; and most preferably pravastatin.

The chemical formulae of the HMG-CoA reductase inhibitors are shown below:

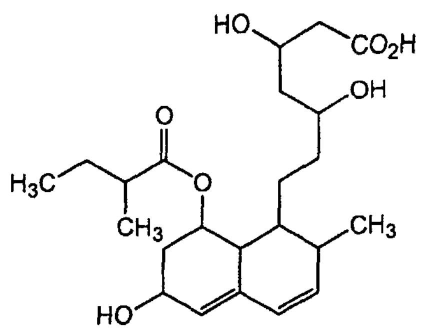

Pravastatin

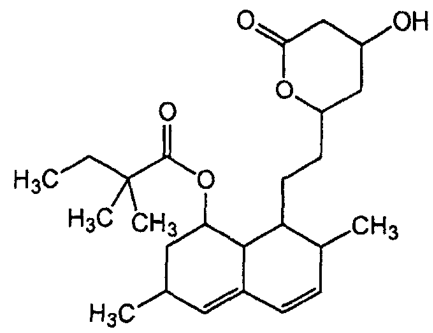

Simvastatin

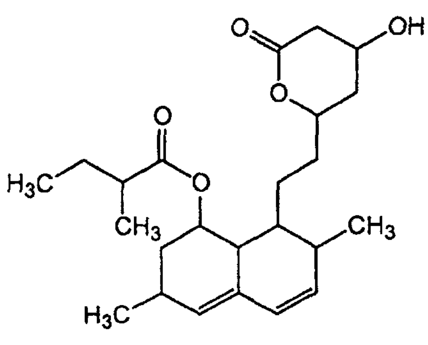

Lovastatin

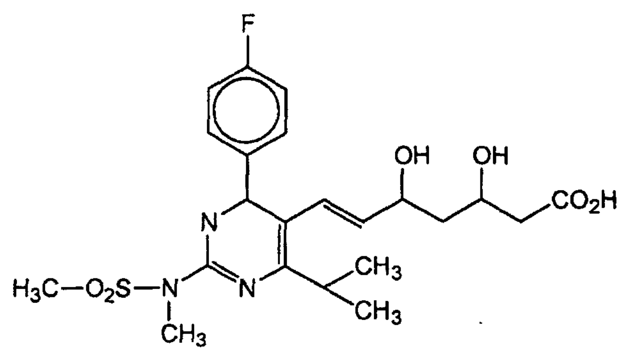

ZD-4522
(Rosuvastatin)

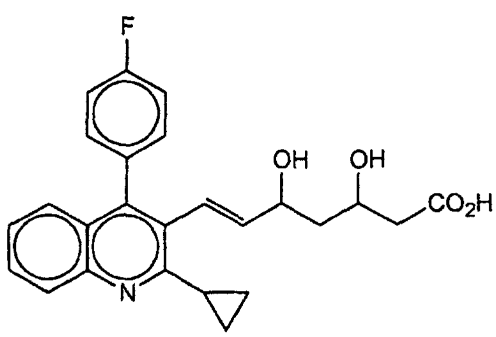

Pitavastatin

Pravastain is disclosed in the specifications of Japanese Patent Application Publication No. Sho 57-2240, U. S. Patent No. 4346227 and the like and is (+)-(3R,5R)-3,5-dihydroxy-7-[(1S, 2S ,6S, 8S, 8aR)-6-hydroxy-2-methyl-8-[(S)-2-methylbutyryloxy]-1,2,6,7,8,8a-hexahydro-1-naphthyl]heptanoic acid. In the present invention the term "pravastatin" is intended to include its lactone ring closure form and its pharmacologically acceptable salts (the sodium salt of pravastatin and the like).

Simvastatin is disclosed in the specifications of Japanese Patent Application Publication No. Sho 56-122375, U.S. Patent No. 4444784 and the like and is (+)-(1S,3R,7S, 8S, 8aR)-1,2,3,7,8,8a-hexahydro-3,7-dimethyl-8-[2-[(2R, 4R)-tetrahydro-4-hydroxy-6-oxo-2H-pyran-2-yl]ethyl]-1-naphthyl] 2,2-dimethylbutylate. In the present invention the term "lovastatin" is intended to include its lactone ring open form and pharmacologically acceptable salts of the lactone ring open form (the sodium salt and the like).

Lovastain is disclosed in the specifications of Japanese Patent Application Publication No. Sho 57-163374, U.S. Patent No. 4231938 and the like and is (+)-(1S, 3R, 7S, 8S, 8aR)-1,2,3,8,8a-hexahydro-3,7-dimethyl-8-[2-[(2R, 4R)-tetrahydro-4-hydroxy-6-oxo-2H-pyran-2-yl]ethyl]-1-naphthyl] (S)-2-methylbutylate. In the present invention the term "lovastatin" is intended to include its lactone ring open form and pharmacologically acceptable salts of the lactone ring open form (the sodium salt and the like).

Pitavastain is disclosed in the specifications of Japanese Patent Application Publication No. Hei 1-279866, U.S. Patent No. 5856336 and the like and is (3R,5S,6E)-7-[2-cyclopropyl-4-(4-fluorophenyl)-3-quinolyl]-3,5-dihydroxy-6-heptanoic acid. In the present invention the term "pitavastatin" is intended to include its lactone ring closed form and its pharmacologically acceptable salts (the calcium salt and the like).

ZD-4522 (Rosuvastatin) is disclosed in the specifications of Japanese Patent Application Publication No. Hei 5-178841, U.S. Patent No. 5260440 and the like and is (+)-(3R,5S)-7-[4-(4-fluorophenyl)-6-isopropyl-2-(N-methyl-N-methanesulfonylamino)pyridin-5-yl]-3,5-dihydroxy-6(E)-heptanoic acid. In the present invention the term "ZD-4522 (Rosuvastatin)" is intended to include its lactone ring closed form and its pharmacologically acceptable salts (the sodium salt, calcium salt and the like).

An active ingredient of the pharmaceutical composition of the present invention is an angiotensin II receptor antagonist. Typical examples of such antagonists are biphenyltetrazole compounds and biphenyl carboxylic acid compounds which are disclosed in the specifications of U.S. Patent No. 5138069, WO Publication No. 91/14679, European Patent Publication No. 433983, U.S. Patent Nos. 5354766, 5196444, 5616599, European Patent Publication No. 502314 and the like; preferably biphenyltetrazole compounds; yet more preferably losartan, irbesartan, valsartan, candesartan, olmesartan or telmisartan; more preferably losartan, irbesartan, valsartan, candesartan or olmesartan; yet more preferably losartan, candesartan or olmesartan; still more preferably losartan or olmesartan; and most preferably olmesartan.

The chemical formulae of typical angiotensin II receptor antagonists are shown below:

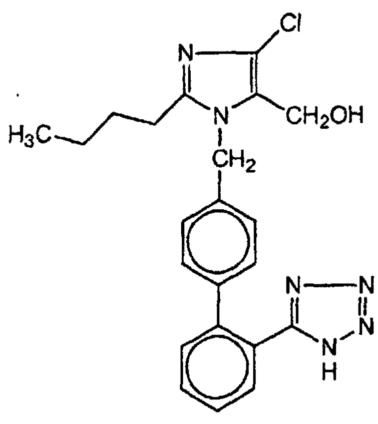

Losartan

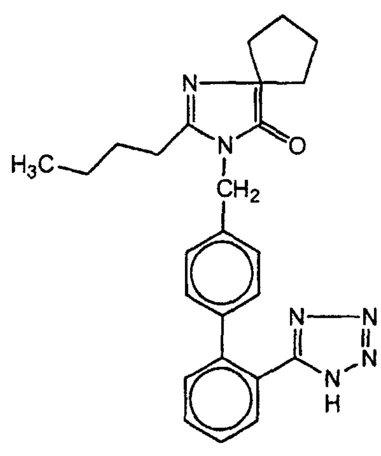

Irbesartan

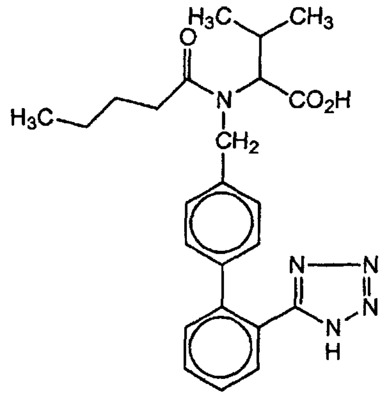

Valsartan

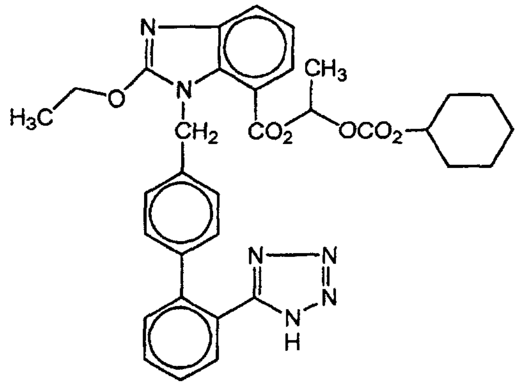

Candesartan

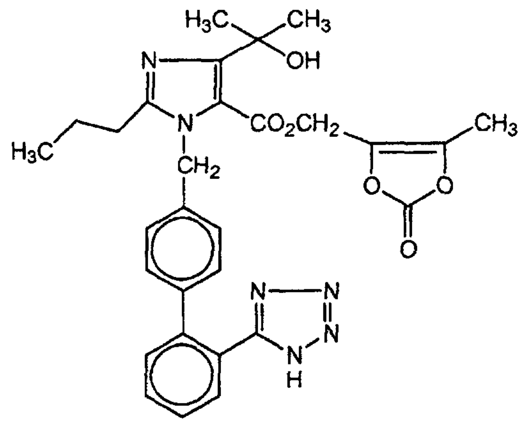

Olmesartan

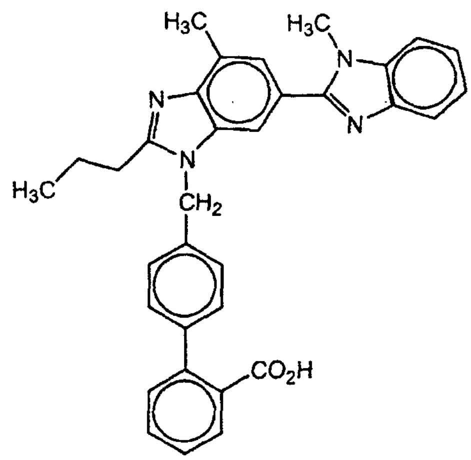

Telmisartan

Losartan (DUP-753) is disclosed in the specifications of Japanese Patent Application Publication No. Sho 63-23868, U.S. Patent No. 5138069 and the like and is 2-butyl-4-chloro-1-[2'-(1 H-tetrazol-5-yl)biphenyl-4-ylmethyl]-1H-imidazole-5-methanol. In the present invention the term "losartan" is intended to include its pharmacologically acceptable salts (losartan potassium and the like).

Irbesartan (SR-47436) is disclosed in the specifications of Japanese Patent Application Publication (Kohyo) No. Hei 4-506222, WO Publication No. 91/14679 and the like and is 2-N-butyl-4-spirocyclopentane-1-[2'-(1H-tetrazol-5-yl)biphenyl-4-ylmethyl]-2-imidazoline-5-one. In the present invention the term "irbesartan" is intended to include its pharmacologically acceptable salts.

Valsartan (CGP-48933) is disclosed in the specifications of Japanese Patent Application Publication No. Hei 4-235149, European Patent Publication No. 433983 and the like and is (S)-N-valeryl-N-[2'-(1H-tetrazol-5-yl)biphenyl-4-ylmethyl]valine. In the present invention the term "valsartan" is intended to include its pharmacologically acceptable esters and salts.

Candesartan (TCV-116) is disclosed in the specifications of Japanese Patent Application Publication No. Hei 4-364171, U.S. Patent No. 5196444 and the like and is I -(cyclohexyloxycarbonyloxy)ethyl 2-ethoxy-1-[2' -(1 H-tetrazol-5-yl)biphenyl-4-ylmethyl]-1H-benzimidazole-7-carboxylate. In the present invention the term "candesartan" is intended to include its carboxylic acid derivatives, pharmacologically acceptable ester derivatives (TCV-116 and the like) of the carboxylic acid derivatives and pharmacologically acceptable salts of candesartan.

Olmesartan (CS-866) is disclosed in the specifications of Japanese Patent Application Publication No. Hei 5-78328, U.S. Patent No. 5616599 and the like and is (5-methyl-2-oxo-1,3-dioxolen-4-yl)methyl 4-(1-hydroxy-1-methylethyl)-2-propyl-1-[2'-(lH-tetrazol-5-yl)biphenyl-4-ylmethyl]imidazole-5-carboxylate. In the present invention the term "olmesartan" is intended to include its carboxylic acid derivatives, pharmacologically acceptable ester derivatives (CS-866 and the like) of the carboxylic acid derivatives and pharmacologically acceptable salts of olmesartan.

Telmisartan (BIBR-277) is disclosed in the specifications of Japanese Patent Application Publication No. Hei 4-346978, U.S. Patent No. 5591762, European Patent Publication No. 502314 and the like and is 4'-[[2-n-propyl-4-methyl-6-(1-methylbenzimidazol-2-yl)-benzimidazol-1-yl]methyl]-biphenyl-2-carboxylate. In the present invention the term "telmisartan" is intended to include its carboxylic acid derivatives, pharmacologically acceptable ester derivatives (BIBR-277 and the like) of the carboxylic acid derivatives and pharmacologically acceptable salts of telmisartan.

An active ingredient of the pharmaceutical composition of the present invention is a calcium channel blocker. Typical examples of such blockers are nifedipine, nicardipine, amlodipine, azelnidipine, and manidipine which are disclosed in the specifications of U.S. Patent Nos. 3485847, 3985758, 4572909, 4772596, 4892875 and the like; preferably nicardipine, amlodipine or azelnidipine; more preferably amlodipine or azelnidipine.

The chemical formulae of the typical calcium channel blockers are shown below:

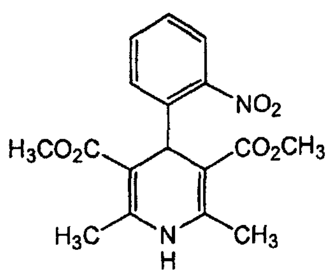

Nifedipine

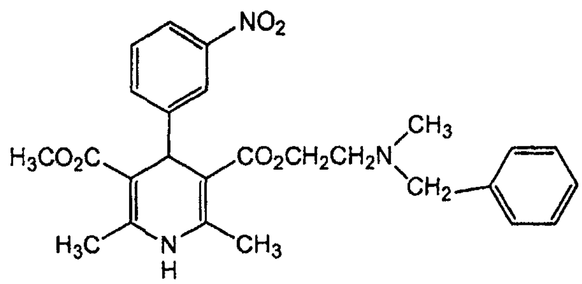

Nicardipine

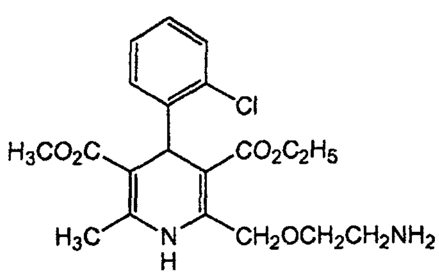

Amlodipine

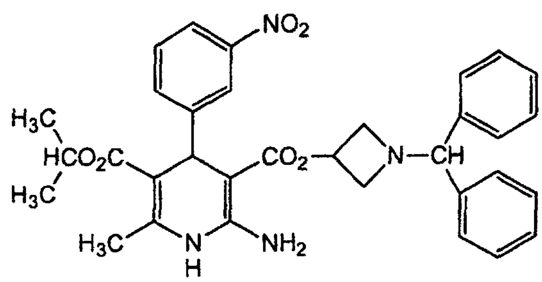

Azelnidipine

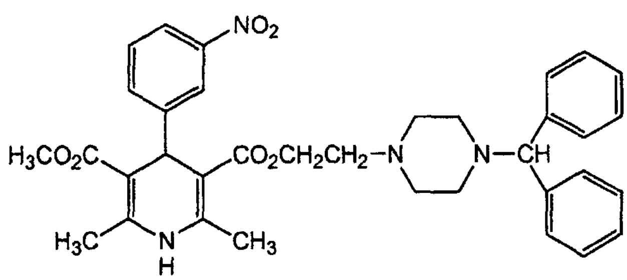

Manidipine

Nifedipine is disclosed in the specifications of U.S. Patent No. 3485847 and the like and is 2,6-dimethyl-3,5-dimethoxycarbonyl-4-(2-nitrophenyl)-1,4-dihydropyridine. In the present invention the term "nifedipine" is intended to include its pharmacologically acceptable salts (the hydrochloride and the like).

Nicardipine is disclosed in the specifications of U.S. Patent No. 3985758, Japanese Patent Application Publication No. Sho 49-108082 and the like and is 2,6-dimethyl-3-[2-(N-benzyl-N-methylamino)ethoxycarbonyl]-5-methoxycarbonyl-4-(3-nitrophenyl)-1,4-dihydropyridine. In the present invention the term "nicardipine" is intended to include its pharmacologically acceptable salts (the hydrochloride and the like).

Amlodipine is disclosed in the specifications of U.S. Patent No. 4572909, Japanese Patent Application Publication No. Sho 58-167569 and the like and is 2-(2-aminoethoxymethyl)-4-(2-chlorophenyl)-3-ethoxycarbonyl-5-methoxycarbonyl-6-methyl-1,4-dihydropyridine. In the present invention the term "amlodipine" is intended to include its pharmacologically acceptable salts (the hydrochloride and the like).

Azelnidipine is disclosed in the specifications of U.S. Patent No. 4772596, Japanese Patent Application Publication No. Sho 63-253082 and the like and is 2-amino-3-(1 -diphenylmethyl-3-azetidinyloxycarbonyl)-5-isopropoxycarbonyl-6-methyl-4-(3-nitrophenyl)-1,4-dihydropyridine. In the present invention the term "azelnidipine" is intended to include its pharmacologically acceptable salts (the hydrochloride and the like).

Manidipine is disclosed in the specifications of U.S. Patent No. 4892875, Japanese Patent Application Publication No. Sho 58-201765 and the like and is 2,6-dimethyl-3-[2-(4-diphenylmethyl-1-piperazinyl)ethoxycarbonyl]-5-methoxycarbonyl-4-(3-nitrophenyl)-1,4-dihydropyridine. In the present invention the term "manidipine" is intended to include its pharmacologically acceptable salts (the hydrochloride and the like).

When the HMG-CoA reductase inhibitors, angiotensin II receptor antagonists and/or calcium channel blockers described above have an asymmetric carbon(s), the present invention encompasses the optical isomers of these compounds and mixtures of said optical isomers. In addition the hydrates of the compounds described above are also encompassed in the present invention.

In the present invention the pharmaceutical composition contains one or more HMG-CoA reductase inhibitors selected from the group consisting of pravastatin, simvastatin, lovastatin, pitavastatin and ZD-4522 and one or more angiotensin receptor antagonists. The pharmaceutical composition of this invention contains one or more HMG-CoA reductase inhibitors and one or more angiotensin receptor antagonists described above and, if necessary, may contain one or more calcium channel blockers.

Preferred pharmaceutical compositions includes:

(1) a pharmaceutical composition wherein the HMG-CoA reductase inhibitor is pravastatin, simvastatin, pitavastatin or ZD-4522;
(2) a pharmaceutical composition wherein the HMG-CoA reductase inhibitor is pravastatin, simvastatin or ZD-4522;
(3) a pharmaceutical composition wherein the HMG-CoA reductase inhibitor is pravastatin or simvastatin;
(4) a pharmaceutical composition wherein the HMG-CoA reductase inhibitor is pravastatin;
(5) a pharmaceutical composition wherein the angiotensin II receptor antagonist as an active ingredient is a biphenyltetrazole compound or a biphenyl carboxylic acid compound;
(6) a pharmaceutical composition wherein the angiotensin II receptor antagonist as an active ingredient is a biphenyltetrazole compound;
(7) a pharmaceutical composition wherein the angiotensin II receptor antagonist as an active ingredient is losartan, irbesartan, valsartan, candesartan, olmesartan or telmisartan;
(8) a pharmaceutical composition wherein the angiotensin II receptor antagonist as an active ingredient is losartan, irbesartan, valsartan, candesartan or olmesartan;
(9) a pharmaceutical composition wherein the angiotensin II receptor antagonist as an active ingredient is losartan, candesartan or olmesartan;
(10) a pharmaceutical composition wherein the angiotensin II receptor antagonist as an active ingredient is losartan or olmesartan;
(11) a pharmaceutical composition wherein the angiotensin II receptor antagonist as an active ingredient is losartan;
(12) a pharmaceutical composition wherein the angiotensin II receptor antagonist as an active ingredient is olmesartan;
(13) a pharmaceutical composition wherein the calcium channel blocker as an active ingredient is nifedipine, nicardipine, amlodipine, azelnidipine or manidipine;
(14) a pharmaceutical composition wherein the calcium channel blocker as an active ingredient is nicardipine, amlodipine or azelnidipine; or
(15) a pharmaceutical composition wherein the calcium channel blocker as an active ingredient is amlodipine or azelnidipine.

Preferred pharmaceutical compositions also includes a pharmaceutical composition that contains a HMG-CoA reductase inhibitor arbitrarily selected from the group consisting of (1), (2), (3) and (4) and an angiotensin II receptor antagonist arbitrarily selected from the group consisting of (5), (6), (7), (8), (9), (10), (11) and (12) and may contain a calcium channel blocker arbitrarily selected from the group consisting of (13), (14) and (15). Examples of such a pharmaceutical composition include, for example:

(16) a pharmaceutical composition wherein the HMG-CoA reductase inhibitor as an active ingredient is pravastain, simvastatin, pitavastatin, or ZD-4522 and the angiotensin II receptor antagonist as an active ingredient is a biphenyltetrazole compound, said pharmaceutical composition optionally further containing a calcium channel blocker as an active ingredient such as nifedipine, nicardipine, amlodipine, azelnidipine or manidipine;
(17) a pharmaceutical composition wherein the HMG-CoA reductase inhibitor as an active ingredient is pravastain, simvastatin, or ZD-4522 and the angiotensin II receptor antagonist as an active ingredient is a biphenyltetrazole

compound, said pharmaceutical composition optionally further containing a calcium channel blocker as an active ingredient such as nifedipine, nicardipine, amlodipine, azelnidipine or manidipine;

(18) a pharmaceutical composition wherein the HMG-CoA reductase inhibitor as an active ingredient is pravastain, simvastatin, pitavastatin, or ZD-4522 and the angiotensin II receptor antagonist as an active ingredient is losartan, irbesartan, valsartan, candesartan, olmesartan or telmisartan, said pharmaceutical composition optionally further containing a calcium channel blocker as an active ingredient such as nifedipine, nicardipine, amlodipine, azelnidipine or manidipine;

(19) a pharmaceutical composition wherein the HMG-CoA reductase inhibitor as an active ingredient is pravastain, simvastatin or ZD-4522 and the angiotensin II receptor antagonist as an active ingredient is losartan, irbesartan, valsartan, candesartan or olmesartan, said pharmaceutical composition optionally further containing a calcium channel blocker as an active ingredient such as nifedipine, nicardipine, amlodipine, azelnidipine or manidipine;

(20) a pharmaceutical composition wherein the HMG-CoA reductase inhibitor as an active ingredient is pravastain or simvastatin and the angiotensin II receptor antagonist as an active ingredient is losartan, candesartan or olmesartan, said pharmaceutical composition optionally further containing a calcium channel blocker as an active ingredient such as nicardipine, amlodipine or azelnidipine;.

(21) a pharmaceutical composition wherein the HMG-CoA reductase inhibitor as an active ingredient is pravastain and the angiotensin II receptor antagonist as an active ingredient is losartan or olmesartan, said pharmaceutical composition optionally further containing a calcium channel blocker as an active ingredient such as amlodipine or azelnidipine; or

(22) a pharmaceutical composition wherein the HMG-CoA reductase inhibitor as an active ingredient is pravastain and the angiotensin II receptor antagonist as an active ingredient is olmesartan, said pharmaceutical composition optionally further containing a calcium channel blocker as an active ingredient such as amlodipine or azelnidipine.

[Advantages of the invention]

The pharmaceutical composition of this invention which contains an HMG-CoA reductase inhibitor selected from the group consisting of pravastain, simvastatin, lovastatin, pitavastatin, and ZD-4522 and an angiotensin II receptor antagonist and, if necessary, may further contain a calcium channel blocker exhibits excellent left ventricular hypertrophy inhibition activity and low toxicity and is a useful pharmaceutical composition for the prevention or treatment of cardiac failure in a warm-blooded animal (particularly a human), or for the prevention of ischemic coronary heart disease or the prevention of the recurrence of ischemic coronary heart disease in a warm-blooded animal (particularly a human).

[Industrial applicability]

The pharmaceutical composition which contains a particular HMG-CoA reductase inhibitor such as pravastatin and the like and a particular angiotensin II acceptor antagonist and, if necessary, may further contain a calcium channel blocker has superior activity to each single pharmaceutical agent alone and to a combined pharmaceutical agent which does not contain a particular HMG-CoA reductase inhibitor such as pravastatin. Such excellent activity can also be obtained even if all these two or three pharmaceutical agents do not exist in the body at the same time. Such activity can be obtained even if the blood concentrations of these pharmaceutical agents are lower to some extent than that obtained on administration at the same time.

According to a presumption, when a pharmaceutical agent of this invention is taken into the living body and reaches its receptor site, it will turn on a switch of the living body. When the blood concentration of said pharmaceutical agent of the pharmaceutical composition becomes very low after the administration of said pharmaceutical agent and said pharmaceutical agent at such low concentration might not exhibit any pharmacological activity, the switch has already been turned on and said pharmaceutical agent of the pharmaceutical composition still exhibits activity for the prevention or treatment of cardiac failure or activity for the prevention of ischemic coronary heart disease or the prevention of the recurrence of ischemic coronary heart disease. Under these conditions, when another pharmaceutical agent(s) is administered, an excellent pharmacological activity can be obtained by the combined effect of its activity for the prevention and treatment of cardiac failure, the prevention of ischemic coronary heart disease or the prevention of the recurrence of ischemic coronary heart disease and the pharmacological effect of the pharmaceutical agent which has already been administered.

Administration of the pharmaceutical agents contained in the pharmaceutical composition at the same time is clinically convenient and a particular HMG-CoA reductase inhibitor such as pravastatin and the like and an angiotensin II acceptor antagonist and, if necessary, a calcium channel blocker can be administered in a unit dosage form which combines these pharmaceutical agents.

When these pharmaceutical agents cannot be blended at the same time in a formulation technique, these

pharmaceutical agents can each be administered in single unit dosage forms of said pharmaceutical agents at the same time or in a single unit dosage form of a pharmaceutical agent and a unit dosage form comprising a combination of the other pharmaceutical agents at the same time.

As mentioned above, the excellent activity of the pharmaceutical composition can be obtained even if these pharmaceutical agents are not administered at the same time. These pharmaceutical agents can each be administered in single unit dosage forms of said pharmaceutical agents at appropriate time intervals after administration of the first unit dosage form or in a single unit dosage form of a pharmaceutical agent and a unit dosage form comprising a combination of the other pharmaceutical agents at appropriate time intervals after administration of the unit dosage form. The maximum time intervals in which the excellent pharmacological activity can be obtained by administration of these pharmaceutical agents can be determined by clinical or animal testing of these pharmaceutical agents.

In general a particular HMG-CoA reductase inhibitor such as pravastatin and the like, an angiotensin II acceptor antagonist and a calcium channel blocker employed in this invention are orally administered. Each of these pharmaceutical agents can be prepared in a single unit dosage form respectively or these pharmaceutical agents are blended and are formulated in a unit dosage form. Examples of such dosage forms include powders, granules, tablets, capsules and the like and these dosage forms can be prepared by conventional formulation techniques. These dosage forms can be prepared, if necessary, using additives such as excipients, lubricating agents, binding agents, disintegrating agents, stabilizing agents, corrigents, diluting agents and the like by techniques known to those skilled in the art.

The excipients are selected from organic excipients, for example, sugar derivatives such as lactose, sucrose, glucose, mannitol and sorbitol; starch derivatives such as corn starch, potato starch, α-starch and dextrin; cellulose derivatives such as crystalline cellulose; acacia; dextran; pullulan; and inorganic excipients, for example, silicate derivatives such as light silicic acid anhydride, synthetic aluminum silicate, calcium silicate and magnesium aluminometasilicate; phosphate derivatives such as calcium hydrogenphosphate; carbonate derivatives such as calcium carbonate; sulfate derivatives such as calcium sulfate; and the like. One or more of the excipients mentioned hereinbefore can be employed in this invention. Preferred excipients are the sugar derivatives and particularly lactose.

The lubricating agents are selected from metal stearates such as stearic acid, calcium stearate and magnesium stearate; talc; colloidal silica; waxes such as beeswax and spermaceti; boric acid; adipic acid; sulfates such as sodium sulfate; glycol; fumaric acid; sodium benzoate; DL-leucine; sodium salts of aliphatic acids; lauryl sulfates such as sodium lauryl sulfate and magnesium lauryl sulfate; silicic acids such as silicic acid anhydride and silicic acid hydrates and starch derivatives as mentioned for the excipients. One or more of the lubricating agents mentioned hereinbefore can be employed in this invention. Preferred lubricating agents are the metal stearates and particularly magnesium stearate.

The binding agents are selected from hydroxypropylcellulose, hydroxypropylmethylcellulose, polyvinylpyrrolidone, macrogol, and compounds as mentioned for excipients. One or more of the binding agents mentioned hereinbefore can be employed in this invention. The preferred binding agents is hydroxypropylcellulose.

The disintegrating agents are selected from cellulose derivatives such as low-substituted hydroxypropylcellulose, carboxymethylcellulose, calcium carboxymethylcellulose, and internally-cross-linked sodium carboxymethylcellulose; and chemically modified starch cellulose derivatives such as carboxymethylstarch, sodium carboxymethylstarch, and cross-linked polyvinylpyrrolidone. One or more of the disintegrating agents mentioned hereinbefore can be employed in this invention. Preferred disintegrating agents are cellulose derivatives and particularly low-substituted hydroxypropylcellulose.

If necessary, additives such as stabilizing agents, corrigents, diluents and the like can be employed in this invention.

The stabilizing agent is selected from para-oxybenzoic acid esters such as methylparaben and propylparaben; alcohols such as chlorobutanol, benzyl alcohol and phenylethyl alcohol; benzalkonium chloride; phenol derivatives such as phenol and cresol; thimerosal; dehydroacetic acid; and sorbic acid. One or more of the stabilizing agents mentioned hereinbefore can be employed in this invention.

The corrigents are, for example, sweetening, souring and flavoring agents which are conventionally used.

The dosage levels and ratios of a particular HMG-CoA reductase inhibitor, an angiotensin II receptor antagonist and a calcium channel blocker will vary depending on various factors such as the pharmacological activity of each of these pharmaceutical agents and the symptoms, age, body weight and the like of the patient. For example, in the case of the angiotensin II receptor antagonist, olmesartan exhibits different activity to losartan in in vivo activity tests using rats with elevated blood pressure. The difference of dosage levels of the two agents is theoretically about one order or more. The dosage level of a particular HMG-CoA reductase inhibitor such as pravastatin, which is usually used as anti-hyperlipidemic agent, for the prevention or treatment of cardiac failure, for the prevention of ischemic coronary heart disease or for the prevention of the recurrence of ischemic coronary heart disease may be less than that for hyperlipidemia. When an angiotensin II receptor antagonist is used together with a HMG-CoA reductase inhibitor, the dosage levels of these pharmaceutical agents may be further lower than those for normal use. When these pharmaceutical agents are used together with a calcium channel blocker, the dosage levels of these pharmaceutical

agents may also be further lower than those for normal use. For example, when pravastatin is used as an anti-hyperlipidemic agent, the dosage level is in the range of 5 - 100 mg/day and when olmesartan is used as an antihypertensive agent, the dosage level is in the range of 0.1 - 200 mg/day; on the other hand when pravastatin and olmesartan is used as an agent for the prevention or treatment of cardiac failure, the prevention of ischemic coronary heart disease or the prevention of the recurrence of ischemic coronary heart disease, the dosage level of pravastatin may be in the range of 1 - 80 mg/day (preferably 10-40 mg/day) and that of olmesartan is in the range of 0.05 - 100 mg/day (preferably 10 - 40 mg/day).

As mentioned hereinbefore, the dosage levels of a particular HMG-CoA reductase inhibitor such as pravastatin and the like, an angiotensin II acceptor antagonist and/or a calcium channel blocker in this invention vary over a wide range; the dosage level of a HMG-CoA reductase inhibitor is in the range of about 1 - 80 mg/day, that of an angiotensin II receptor is in the range of about 0.05-100 mg/day and that of a calcium channel blocker is in the range of about 1-100 mg/day.

The dosage ratio of these pharmaceutical agents will vary over a wide range; the general dosage ratio (by weight) of a particular HMG-CoA reductase inhibitor such as pravastatin, an angiotensin II receptor antagonist and/or a calcium channel blocker is in the range between 1:500:500 and 500:1:1.

The dosage levels of these pharmaceutical agents mentioned hereinbefore are administered once throughout the day at the same time or are separately administered respectively at appropriate time intervals once throughout the day. The dosage levels of these pharmaceutical agents mentioned hereinbefore are divided to form several subunits respectively. The subunits are administered several times throughout the day at the same time or are separately administered respectively at appropriate time intervals several times throughout the day.

[Best mode for carrying out the invention]

The present invention is further illustrated by Examples and Formulation examples, however the scope of the present invention is not limited to these examples.

Test Example 1

Ameliorating effects of the compounds on left ventricular hypertrophy.

Forty patients with hypertension and hyperlipidemia whose average diastolic and systolic blood pressures and total plasma cholesterol levels calculated from 3 determinations were 95-115 mmHg (DBP), 160-200 mmHg, and higher than 240 mg/dl, respectively were equally divided into 2 groups consisting of 20 patients per group; group 1-I (m/f: 14/6, average age, height and weight were 61 years, 166 cm, and 65 kg, respectively) and group 1-II (m/f: 14/6, average age, height, and weight were 64 years, 164 cm, and 64 kg, respectively). A further 20 hypertensive patients (m/f: 15/5) with average blood pressure as described above and normal cholesterol levels were recruited into a group 1-III (average age, height, and weight were 62 years, 165 cm, and 66 kg, respectively). Lastly, in order to obtain the normal mass of the left ventricle, 10 healthy volunteer subjects (m/f: 7/3; 60 years old, 168 cm and 70 kg) with normal blood pressure (> 140/90 mmHg) and normal plasma cholesterol levels were recruited into a control group.

Pravastatin (10 mg), losartan (50 mg) and amlodipine (5 mg) were concomitantly administered to patients in the 1-I group, once daily for 6 months. Patients in the 1-II group received losartan (50 mg) and amlodipine (5 mg) concomitantly and orally once daily and underwent diet therapy for 6 months. Patients in the 1-III group received losartan (50 mg) and amlodipine (5 mg) concomitantly once daily for 6 months.

In all subjects, the mass of the left ventricle (LV mass) and the mass index of the left ventricle (LV mass index) were calculated from the diameter of the left ventricle in diastolic phase (LV end-diastolic dimension; EDD), the ventricular septum thickness (VS), and the posterior wall thickness (PW) which were determined by Doppler color flowmapping methods using a two-dimension echo-cardiography device (Hewlett-Packard, SONOS 2,000) and according to the following equation:

$$\text{LV mass (g)}=0.80\text{x}\{1.04\text{x}[(\text{EDD}+\text{VS}+\text{PW})^3-\text{EDD}^3]\}+0.6$$

$$\text{LV mass index} = \text{LV mass}/(\text{area of the body surface, g/cm}^2)$$

The calculated LV mass indexes are presented in Table 1. The LV mass index in the control group was 83±10 $g/m^2$ throughout the study.

[Table 1]

| | LV mass Index ($g/m^2$) | |
|---|---|---|
| Group | Before treatment | After treatment |
| 1-I | 143 ±12 | 107 ±13 |
| 1-II | 142 ± 18 | 122 ± 11 |
| 1-III | 142 ± 18 | 122 ± 11 |

Following treatment, the LV masses were reduced by 24%, 13%, and 13% in the 1-I (treated with pravastatin, losartan and amlodipine), 1-II (treated with diet therapy, losartan and amlodipine), and 1-III groups (losartan and amlodipine), respectively. Thus, patients in group 1-I (treated with pravastatin, losartan and amlodipine) showed the biggest reduction in LV mass.

Similarly to the results observed in test example 1, treatment with either a combination of pravastatin (10 mg) and losartan (50 mg) or of pravastatin (10 mg) with olmesartan (5 mg) shows excellent retraction in LV mass.

Furthermore, combination therapy of either pravastatin plus olmesartan or simvastatin plus olmesartan ameliorated LV hypertrophy in a stress-loaded LV hypertrophy model in rats.

Test Example 2

Suppression of hypertrophy of ventricular cells in the area adjacent to infarction.

(Preparation of the animal model)

Adult male Wistar rats weighing 250-300 g (normal plasma cholesterol levels) were used. Animals received a normal-sodium diet containing 0.32 wt% sodium prior to and throughout the experiment. Water was taken *ad libitum.* Five rats/cage were placed and bred in a breeding room controlled at a stable room temperature (22±1°C) and humidity and with a standard light-darkness cycle. Echocardiography was recorded through the esophagus 24 hr after ligation of the anterior descending branch of the left coronary artery (day 0), then the animals were randomly divided into the following 4 groups consisting of 10 animals per group: control group (vehicle administration), 2-I group (administration of pravastatin 5 mg/kg/day in drink-water), 2-II group (administration of CS-866 2 mg/kg/day), and 2-III group (pravastatin 5 mg/kg/day + CS-866 2 mg/kg/day).

Surgical procedures were carried out as follows: rats were anesthetized with an intraperitoneal injection of ketamine (90 mg/kg). Under anesthesia, air was inspired into the animals by an artificial respirator for small animals (Model 683, Harvard Apparatus, Boston, MA) through a polyethylene catheter of gauge 14. Left thoracotomy was performed and the heart exposed. The anterior descending branch of the left coronary artery was ligated with a silk thread (6-0) between the bifurcation of the pulmonary artery and the left atrium. Then the muscle and the skin were sewn to close the chest. The rats of the normal group underwent a similar procedure except for the ligation of the coronary artery, in which the thread was passed through beneath the coronary artery. Drugs were administered to the rats for 4 weeks starting on the day when they were grouped.

(Determination of the ventricular cells in adjacent areas to the infarction)

The rats were heparinized and the hearts were removed. The heart was warmed at 37°C, and perfused by modified Langendorff's method at a constant flow-rate of 8 ml/min. The cardiac and non-cardiac muscle cells were enzymatically separated using collagenase according to previously described methods (Stewart et al., 1994). For the perfusion fluid, oxygenated Krebs-Henseleit buffer solution (pH: 7.4) containing 138 mmol NaCl, 4.7 mmol KCl, 1.5 mmol $CaCl_2$, 1.2 mmol $MgCl_2$, 10.0 mmol glucose, 10.0 mmol pyruvic acid, 5 mmol HEPES, and 20 U/l insulin was used. After the solution described above was perfused for 5 min to equilibrate, the perfusing solution was changed to Krebs-Henseleit solution not containing $Ca^{2+}$, and further perfused for five minutes. Then collagenase B (1 mg/ml, Boehringer Mannheim Corp., Indianapolis, IN, USA) was added to the perfusing solution. After perfusion with the solution containing collagenase B for 10-15 min, the polyethylene cannula was removed from the heart and the non-digested infarction area removed. The left ventricle involving the ventricular septum was recovered and dissected in oxygenated Kraft-Brule buffer solution (pH: 7.2) containing 70 mmol glutamic acid, 25 mmol KCl, 10 mmol $K_2HPO_4$, 10 mmol oxalic acid, 10 mmol taurine, 11 mmol glucose, 2 mmol pyruvic acid, 2 mmol ATP, 2 mmol creatine phosphate, 10 mmol HEPES and 5 mmol $MgCl_2$. The obtained cell suspension solution was filtrated to remove the massive tissue. The mass of the cardiac cells in the adjacent area to the infarction was traced for determination under a phase-contrast

microscope. The obtained masses of the cardiac muscle cells in adjacent areas to the infarction are presented in Table 2.

Table 2

| Group | Mass of cardiac muscle cells in adjacent area to the infarction (µm2) |
|---|---|
| normal | 2,737 |
| control | 3,812 |
| 2-I group | 3,053 |
| 2-II group | 2,940 |
| 2-III group | 2,442 |

From the present results, the size of the cardiac muscle cells in the adjacent area to the infarction was retracted by 760 $\mu m^2$ in group 2-I (pravastatin-administered group), 872 $\mu m^2$ in group 2-II (olmesartan-administered group), and 1,370 $\mu m^2$ in group 2-III (pravastatin plus olmesartan-administered group) versus control rats. Thus the most significant ameliorating effects were observed in group 2-III (pravastatin plus olmesartan administered group). It is clear that combined administration of olmesartan and pravastatin significantly retracts the size of the affected cardiac muscle cells versus each compound used alone, and furthermore, combined administration decreases the sizes of the cells more significantly than that observed in the normal group.

| Formulation example 1 | tablet |
|---|---|
| losartan | 50.0 mg |
| pravastatin sodium salt | 10.0 mg |
| amlodipine | 5.0 mg |
| lactose | 108.0 mg |
| corn starch | 25.0 mg |
| magnesium stearate | 2.0mg |
| | 200.0 mg |

The powdery ingredients listed above are blended and tabletted using a tablet machine to give a tablet (200 mg).

| Formulation example 2 | tablet |
|---|---|
| losartan | 50.0 mg |
| pravastatin sodium salt | 10.0 mg |
| lactose | 113.0 mg |
| corn starch | 25.0 mg |
| magnesium stearate | 2.0mg |
| | 200.0 mg |

The powdery ingredients listed above are blended and tabletted using a tablet machine to give a tablet (200 mg).

| Formulation example 3 | tablet |
|---|---|
| olmesartan | 5.0 mg |
| pravastatin sodium salt | 10.0 mg |
| lactose | 158.0 mg |
| corn starch | 25.0 mg |
| magnesium stearate | 2.0 mg |
| | 200.0 mg |

The powdery ingredients listed above are blended and tabletted using a tablet machine to give a tablet (200

mg).

**Claims**

1. A pharmaceutical composition for the prevention or treatment of cardiac failure, said pharmaceutical composition containing a HMG-CoA reductase inhibitor selected from the group consisting of pravastatin, simvastatin, lovastatin, pitavastatin and ZD-4522 and an angiotensin II receptor antagonist and optionally further containing a calcium channel blocker.

2. A pharmaceutical composition according to claim 1 wherein the HMG-CoA reductase inhibitor is pravastatin, simvastatin, pitavastatin or ZD-4522.

3. A pharmaceutical composition according to claim 1 wherein the HMG-CoA reductase inhibitor is pravastatin, simvastatin or ZD-4522.

4. A pharmaceutical composition according to claim 1 wherein the HMG-CoA reductase inhibitor is pravastatin or simvastatin.

5. A pharmaceutical composition according to claim 1 wherein the HMG-CoA reductase inhibitor is pravastatin.

6. A pharmaceutical composition for the prevention of ischemic coronary heart disease or for the prevention of the recurrence of ischemic coronary heart disease, said pharmaceutical composition containing a HMG-CoA reductase inhibitor selected from the group consisting of pravastatin, simvastatin, lovastatin, pitavastatin and ZD-4522 and an angiotensin II receptor antagonist and optionally further containing a calcium channel blocker.

7. A pharmaceutical composition according to claim 6 wherein the HMG-CoA reductase inhibitor is pravastatin, simvastatin, pitavastatin or ZD-4522.

8. A pharmaceutical composition according to claim 6 wherein the HMG-CoA reductase inhibitor is pravastatin, simvastatin or ZD-4522.

9. A pharmaceutical composition according to claim 6 wherein the HMG-CoA reductase inhibitor is pravastatin or simvastatin.

10. A pharmaceutical composition according to claim 6 wherein the HMG-CoA reductase inhibitor is pravastatin.

11. A method for the prevention or treatment of cardiac failure, said method comprising administering to a warm-blooded animal in need of such prevention or treatment a pharmacologically effective amount of a pharmaceutical composition that contains a HMG-CoA reductase inhibitor selected from the group consisting of pravastatin, simvastatin, lovastatin, pitavastatin and ZD-4522 and an angiotensin II receptor antagonist and optionally further contains a calcium channel blocker.

12. A method according to claim 11 wherein the HMG-CoA reductase inhibitor is pravastatin, simvastatin, pitavastatin or ZD-4522.

13. A method according to claim 11 wherein the HMG-CoA reductase inhibitor is pravastatin, simvastatin or ZD-4522.

14. A method according to claim 11 wherein the HMG-CoA reductase inhibitor is pravastatin or simvastatin.

15. A method according to claim 11 wherein the HMG-CoA reductase inhibitor is pravastatin.

16. A method for the prevention of ischemic coronary heart disease or the prevention of the recurrence of ischemic coronary heart disease, said method comprising administering to a warm-blooded animal in need of such prevention a pharmacologically effective amount of a pharmaceutical composition that contains a HMG-CoA reductase inhibitor selected from the group consisting of pravastatin, simvastatin, lovastatin, pitavastatin and ZD-4522 and an angiotensin II receptor antagonist and optionally further contains a calcium channel blocker.

**17.** A method according to claim 16 wherein the HMG-CoA reductase inhibitor is pravastatin, simvastatin, pitavastatin or ZD-4522.

**18.** A method according to claim 16 wherein the HMG-CoA reductase inhibitor is pravastatin, simvastatin or ZD-4522.

**19.** A method according to claim 16 wherein the HMG-CoA reductase inhibitor is pravastatin or simvastatin.

**20.** A method according to claim 16 wherein the HMG-CoA reductase inhibitor is pravastatin.

## INTERNATIONAL SEARCH REPORT

International application No.
PCT/JP01/07437

**A. CLASSIFICATION OF SUBJECT MATTER**
Int.Cl[7] A61K31/404, 31/22, 31/366, 31/41, 31/4184, 31/505, 31/47, 45/06, A61P9/10, 9/04, 43/00

According to International Patent Classification (IPC) or to both national classification and IPC

**B. FIELDS SEARCHED**

Minimum documentation searched (classification system followed by classification symbols)
Int.Cl7 A61K31/404, 31/22, 31/366, 31/41, 31/4184, 31/505, 31/47, 45/06,A61P9/10, 9/04, 43/00

Documentation searched other than minimum documentation to the extent that such documents are included in the fields searched

Electronic data base consulted during the international search (name of data base and, where practicable, search terms used)
CAPLUS(STN), MEDLINE(STN), EMBASE(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 97/37688 A2 (TAKEDA CHEMICAL INDUSTRIES LTD.), 16 October, 1997 (16.10.97), & EP 914158 A2 & US 6107323 A & JP 9-323940 A | 1-10<br>1-10 |
| Y | WO 99/11260 A1 (PFIZER INC.), 11 March, 1999 (11.03.99), & EP 1009400 A1 & JP 2001-514223 A | 1-10 |
| Y | WO 95/26188 A1 (MERCK AND CO. INC.), 05 October, 1995 (05.10.95), & EP 754042 A1 & US 5663186 A & US 5663187 A & JP 9-510973 A | 1-10 |
| Y | WO 99/11263 A1 (PFIZER INC.), 11 March, 1999 (11.03.99), & EP 1003507 A1 & JP 2001-514224 A | 1-10 |
| Y | WO 99/11259 A1 (PFIZER INC.), 11 March, 1999 (11.03.99), & EP 1003503 A1 & JP 2001-514222 A | 1-10 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 November, 2001 (06.11.01) | 20 November, 2001 (20.11.01) |
| Name and mailing address of the ISA/<br>Japanese Patent Office | Authorized officer |
| Facsimile No. | Telephone No. |

**INTERNATIONAL SEARCH REPORT**

International application No.
PCT/JP01/07437

C (Continuation). DOCUMENTS CONSIDERED TO BE RELEVANT

| Category* | Citation of document, with indication, where appropriate, of the relevant passages | Relevant to claim No. |
|---|---|---|
| Y | JP 10-81633 A2 (SANKYO COMPANY, LIMITED), 31 March, 1998 (31.03.98), (No family) | 1-10 |
| P,X | WO 01/15674 A2 (AVENTIS PHARMA DEUTSCHLAND GMBH), 08 March, 2001 (08.03.01), (No family) | 1-10 |

**INTERNATIONAL SEARCH REPORT**

International application No.
PCT/JP01/07437

**Box I Observations where certain claims were found unsearchable (Continuation of item 1 of first sheet)**

This international search report has not been established in respect of certain claims under Article 17(2)(a) for the following reasons:

1. ☒ Claims Nos.: 11-20
because they relate to subject matter not required to be searched by this Authority, namely:

Claims 11 to 20 pertain to methods for treatment of the human body by therapy and thus relate to a subject matter which this International Searching Authority is not required, under the provisions of Article 17(2)(a)(i) of the PCT and Rule 39(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)